# EUROPEAN PATENT SPECIFICATION

(11) **EP 1 597 353 B1**
(45) Date of publication and mention of the grant of the patent: **24.11.2010**
(21) Application number: 04715133.7
(22) Date of filing: 26.02.2004
(51) Int. Cl.: C12N 1/00, G01N 33/48, G01N 33/53, G01N 33/574

(54) **CIRCULATING TUMOR CELLS (CTC's): EARLY ASSESSMENT OF TIME TO PROGRESSION SURVIVAL AND RESPONSE TO THERAPY IN METASTATIC CANCER PATIENTS**
ZIRKULIERENDE TUMORZELLEN (CTC's): FRÜHE BEURTEILUNG VON TIME-TO-PROGRESSION, ÜBERLEBEN UND ANSPRECHEN AUF THERAPIE BEI PATIENTEN MIT METASTASIERTEM KREBS
CELLULES TUMORALES CIRCULANTES (CTC): EVALUATION PRECOCE DU TEMPS D'EVOLUTION, DE LA SURVIE ET DE LA REACTION AUX THERAPIES DES PATIENTS CANCEREUX METASTASIQUES

(30) Priority: 27.02.2003 US 450519 P; 25.11.2003 US 524759 P
(43) Date of publication of application: 23.11.2005
(73) Proprietor: Veridex, LLC, North Raritan NJ 08869 (US)
(72) Inventor: ALLARD, Jeffrey, W., North Wales, PA 19454 (US); CRISTOFANILLI, Massimo, Pearland, TX 77584 (US); TERSTAPPEN, Leon, W., M., M., Huntingdon Valley, PA 19006 (US)
(74) Representative: Fisher, Adrian John
(86) International application number: PCT/US2004/005848
(87) International publication number: WO 2004/076643

(56) References cited:
- WO-A1-99/41613
- US-A- 5 993 388
- US-A1- 2002 098 535
- US-A1- 2002 098 535
- US-A1- 2002 172 987
- KAGAN M ET AL: "A SAMPLE PREPARATION AND ANALYSIS SYSTEM FOR IDENTIFICATION OF CIRCULATING TUMOR CELLS" JOURNAL OF CLINICAL LIGAND ASSAY, CLINICAL LIGAND ASSAY SOCIETY, WAYNE, MI, US, vol. 25, no. 1, 21 March 2002 (2002-03-21), pages 104-110, XP008052823 ISSN: 1081-1672
- WITZIG THOMAS E ET AL: "Detection of circulating cytokeratin-positive cells in the blood of breast cancer patients using immunomagnetic enrichment and digital microscopy." CLINICAL CANCER RESEARCH : AN OFFICIAL JOURNAL OF THE AMERICAN ASSOCIATION FOR CANCER RESEARCH MAY 2002, vol. 8, no. 5, May 2002 (2002-05), pages 1085-1091, XP002451219 ISSN: 1078-0432
- PITUCH-NOWOROLSKA ANNA ET AL: "Evaluation of circulating tumour cells expressing CD44 variants in the blood of gastric cancer patients by flow cytometry" ANTICANCER RESEARCH, vol. 18, no. 5B, September 1998 (1998-09), pages 3747-3752, XP008083618 ISSN: 0250-7005
- SABILE A ET AL: "Efficiency of Ber-EP4 antibody for isolating circulating epithelial tumor cells before RT-PCR detection" AMERICAN JOURNAL OF CLINICAL PATHOLOGY, PHILADELPHIA, PA, US, vol. 112, no. 2, August 1999 (1999-08), pages 171-178, XP009028763 ISSN: 0002-9173
- DOUDINA OLGA ET AL: "Circulating tumor cells in patients with localized breast cancer" BLOOD, vol. 98, no. 11 Part 2, 16 November 2001 (2001-11-16), page 341b, XP008083708 & 43RD ANNUAL MEETING OF THE AMERICAN SOCIETY OF HEMATOLOGY, PART 2; ORLANDO, FLORIDA, USA; DECEMBER 07-11, 2001 ISSN: 0006-4971
- PACHMANN K ET AL: "REAL TIME MONITORING OF THE EFFICACY OF ADJUVANT THERAPY IN BREAST CANCER QUANTIFYING THE REDUCTION OF CIRCULATING TUMOR CELLS BY MAINTRAC (LASER SCANNING CYTOMETRY OF MAGNETIC BEAD ENRICHED CELLS)" BREAST CANCER RESEARCH AND TREATMENT, NIJHOFF, BOSTON, US, vol. 64, no. 1, November 2000 (2000-11), page 96, XP009017863 ISSN: 0167-6806

## Description

### Background

### • Field of the Invention

The invention relates generally to cancer prognosis and survival In metastatic cancer patients, based on the presence of morphologically intact circulating cancer cells (CTC) in blood. More specifically, diagnostic methods, reagents and apparatus are described that correlate the presence of circulating cancer cells in 7.5 ml of blood of metastatic breast cancer patients with time to disease progression and survivability. Circulating tumor cells are determined by highly sensitive methodologies capable of isolating and imaging 1 or 2 cancer cells in approximately 5 to 50 ml of peripheral blood, the level of the tumor cell number and an increase in tumor cell number during treatment are correlated to the time to progression, time to death and response to therapy.

### • Background Art

Despite efforts to improve treatment and management of cancer patients, survival in cancer patients has not improved over the past two decades for many cancer types. Accordingly, most cancer patients are not killed by their primary tumor, but they succumb instead to metastases: multiple widespread tumor colonies established by malignant cells that detach themselves from the original tumor and travel through the body, often to distant sites. The most successful therapeutic strategy in cancer is early detection and surgical removal of the tumor while still organ confined. Early detection of cancer has proven feasible for some cancers, particularly where appropriate diagnostic tests exist such as PAP smears in cervical cancer, mammography in breast cancer, and serum prostate specific antigen (PSA) in prostate cancer. However, many cancers detected at early stages have established micrometastases prior to surgical resection. Thus, early and accurate determination of the cancer's malignant potential is important for selection of proper therapy.

Optimal therapy will be based on a combination of diagnostic and prognostic information. An accurate and reproducible diagnostic test is needed to provide specific information regarding the metastatic nature of a particular cancer, together with a prognostic assessment that will provide specific information regarding survival.

A properly designed prognostic test will give physicians information about risk and likelihood of survival, which in turn gives the patient the benefit of not having to endure unnecessary treatment. Patient morale would also be boosted from the knowledge that a selected therapy will be effective based on a prognostic test. The cost savings associated with such a test could be significant as the physician would be provided with a rationale for replacing ineffective therapies. A properly developed diagnostic and prognostic data bank in the treatment and detection of metastatic cancer focusing on survival obviously would provide an enormous benefit to medicine (US 6,063,586).

If a primary tumor is detected early enough, it can often be eliminated by surgery, radiation, or chemotherapy or some combination of those treatments. Unfortunately, the metastatic colonies are difficult to detect and eliminate and it is often impossible to treat all of them successfully. Therefore from a clinical point of view, metastasis can be considered the conclusive event in the natural progression of cancer. Moreover, the ability to metastasize is a property that uniquely characterizes a malignant tumor.

### Soluble Tumor Antigen:

Based on the complexity of cancer and cancer metastasis and the frustration in treating cancer patients over the years, many attempts have been made to develop diagnostic tests to guide treatment and monitor the effects of such treatment on metastasis or relapse.

One of the first attempts to develop a useful test for diagnostic oncology was the formulation of an immunoassay for carcinoembryonic antigen (CEA). This antigen appears on fetal cells and reappears on tumor cells in certain cancers. Extensive efforts have been made to evaluate the usefulness of testing for CEA as well as many other "tumor" antigens, such as prostate specific antigen (PSA), CA 15.3, CA 125, prostate-specific membrane antigen (PSMA), CA 27.29, p27 found in either tissue samples or blood as soluble cellular debris. These and other debris antigens are thought to be derived from destruction of both circulating and non-circulating tumor cells, and thus their presence may not always reflect metastatic potential, especially if the cells rupture while in an apoptotic state.

Additional tests used to predict tumor progression in cancer patients have focused upon correlating enzymatic indices like telomerase activity in biopsy-harvested tumor samples with an indication of an unfavorable or favorable prognosis (US 5,693,474; US 5,639,613). Assessing enzyme activity in this type of analysis can involve time-consuming laboratory procedures such as gel electrophoresis and Western blot analysis. Also, there are variations in the signal to noise and sensitivity in sample analysis based on the origin of the tumor. Despite these shortcomings, specific soluble tumor markers in blood can provide a rapid and efficient approach for developing a therapeutic strategy early in treatment. For example, detection of serum HER-2/neu and serum CA 15-3 in patients with metastatic breast cancer have been shown to be prognostic factors for metastatic breast cancer (Ali S.M., Leitzel K., Chinchilli V.M., Engle L., Demers L., Harvey H.A., Carney W., Allard J.W. and Lipton A., Relationship of Serum HER-2/neu and Serum CA 15-3 in Patients with Metastatic Breast Cancer, Clinical Chemistry, 48(8):1314-1320 (2002)). Increased HER-2/neu results in decreased response to hormone therapy, and is a significant prognostic factor in predicting responses to hormone receptor-positive metastatic breast cancer. Thus in malignancies where the HER-2/neu oncogene product is associated, methods have been described to monitor therapy or assess risks based on elevated levels (US 5,876,712). However in both cases, the base levels during remission, or even in healthy normals, are relatively high and may overlap with concentrations found in patients, thus requiring multiple testing and monitoring to establish patient-dependent baseline and cut-off levels.

In prostate cancer, PSA levels in serum have proven to be useful in early detection. When used with a follow-up physical examination (digital rectal exam) and biopsy, the PSA test has improved detection of prostate cancer at an early stage when it is best treated.

However, PSA or the related PSMA testing leaves much to be desired. For example, elevated levels of PSA weakly correlate with disease stage and appear not to be a reliable indicator of the metastatic potential of the tumor. This may be due in part to the fact that PSA is a component of normal prostate tissue and benign prostatic hyperplasia (BHP) tissue. Moreover, approximately 30% of patients with alleged localized prostate cancer and corresponding low serum PSA concentrations, may have metastatic disease (Moreno et al., Cancer Research, 52:6110 (1992)).

### Genetic markers:

One approach for determining the presence of malignant prostate tumor cells has been to test for the expression of messenger RNA from PSA in blood. This is being done through the laborious procedure of isolating all of the mRNA from the blood sample and performing reverse transcriptase PCR. No significant correlation has been described between the presence of shed tumor cells in blood and the ability to identify which patients would benefit from more vigorous treatment (Gomella LG., J of Urology, 158:326-337 (1997)). Additionally, false positives are often observed using this technique. There is an added drawback, which is that there is a finite and practical limit to the sensitivity of this technique based on the sample size. Typically, the test is performed on 10⁵ to 10⁶ cells separated from interfering red blood cells, corresponding to a practical lower limit of sensitivity of one tumor cell/0.1 ml of blood (about 10 tumor cells in one ml of blood) before a signal is detected. Higher sensitivity has been suggested by detecting hK2 RNA in tumor cells isolated from blood (US 6,479,263; US 6,235,486).

Qualitative RT-PCR based studies with blood-based nucleotide markers has been used to indicate that the potential for disease-free survival for patients with positive CEA mRNA in pre-operative blood is worse than that of patients negative for CEA mRNA (Hardingham J.E., Hewett P.J., Sage R.E., Finch J.L., Nuttal J.D., Kotasel D. and Dovrovic A., Molecular detection of blood-borne epithelial cells in colorectal cancer patients and in patients with benign bowel disease, Int. J. Cancer 89:8-13 (2000): Taniguchi T., Makino M., Suzuki K., Kaibara N., Prognostic significance of reverse transcriptase-polymerase chain reaction measurement of carcinoembryonic antigen mRNA levels in tumor drainage blood and peripheral blood of patients with colorectal carcinoma, Cancer 89:970-976 (2000)). The prognostic value of this endpoint is dependent upon CEA mRNA levels, which are also induced in healthy individuals by G-CSF, cytokines, steroids, or environmental factors. Hence, the CEA mRNA marker lacks specificity and is clearly not unique to circulating colorectal cancer cells. Other reports have implicated tyrosinase mRNA in peripheral blood and bone marrow as a marker for malignant melanoma in stage II-IV patients (Ghossein R.A., Coit D., Brennan M., Zhang Z.F., Wang Y., Bhattacharya S., Houghton A., and Rosai J., Prognostic significance of peripheral blood and bone marrow tyrosinase messenger RNA in malignant melanoma, Clin Cancer Res., 4(2):419-428 (1998)). Again using tyrosinase mRNA as a soluble tumor marker is subject to the previously cited limitations of soluble tumor antigens as indicators of metastatic potential and patient survival.

The aforementioned and other studies, while seemingly prognostic under the experimental conditions, do not provide for consistent data with a long follow-up period or at a satisfactory specificity. Accordingly, these efforts have proven to be somewhat futile as the appearance of mRNA for antigens in blood have not been generally predictive for most cancers and are often detected when there is little hope for the patient.

In spite of this, real-time reverse transcriptase-polymerase chain reaction (RT-PCR) has been the only procedure reported to correlate the quantitative detection of circulating tumor cells with patient prognosis. Real-time RT-PCR has been used for quantifying CEA mRNA in peripheral blood of colorectal cancer patients (Ito S., Nakanishi H., Hirai T., Kato T., Kodera Y., Feng Z., Kasai Y., Ito K., Akiyama S., Nakao A., and Tatematsu M., Quantitative detection of CEA expressing free tumor cells in the peripheral blood of colorectal cancer patients during surgery with real-time RT-PCR on a Light Cycler, Cancer Letters, 183:195-203 (2002)). Using Kaplan-Meier type analysis, disease free survival of patients with positive CEA mRNA in post-operative blood was significantly shorter than in cases that were negative for CEA mRNA. These results suggest that tumor cells were shed into the bloodstream (possibly during surgical procedures or from micro metastases already existing at the time of the operation), and resulted in poor patient outcomes in patients with colorectal cancer. The sensitivity of this assay provided a reproducibly detectable range similar in sensitivity to conventional RT-PCR. As mentioned, these detection ranges are based on unreliable conversions of amplified product to the number of tumor cells. The extrapolated cell count may include damaged CTC incapable of metastatic proliferation. Further, PCR-based assays are limited by possible sample contamination, along with an inability to quantify tumor cells. Most importantly, methods based on PCR, flowcytometry, cytoplasmic enzymes and circulating tumor antigens cannot provide essential morphological information confirming the structural integrity underlying metastatic potential of the presumed CTC and thus constitute functionally less reliable surrogate assays than the highly sensitive imaging methods embodied, in part, in this invention.

### Assessment of intact tumor cells in cancer detection and prognosis:

Detection of intact tumor cells in blood provides a direct link to recurrent metastatic disease in cancer patients who have undergone resection of their primary tumor. Unfortunately, the same spreading of malignant cells continues to be missed by conventional tumor staging procedures. Recent studies have shown that the presence of a single carcinoma cell in the bone marrow of cancer patients is an independent prognostic factor for metastatic relapse (Dial IJ, Kaufman M, Goerner R, Costa SD, Kaul S, Bastert G. Detection of tumor cells in bone marrow of patients with primary breast cancer: a prognostic factor for distant metastasis. J Clin Oncol, 10:1534-1539, 1992). But these invasive techniques are deemed undesirable or unacceptable for routine or multiple clinical assays compared to detection of disseminated epithelial tumor cells in blood.

An alternative approach incorporates immunomagnetic separation technology and provides greater sensitivity and specificity in the unequivocal detection of intact circulating cancer cells. This simple and sensitive diagnostic tool, as described (US6,365,362; US6,551,843; US6,623,982; US6,620,627; US6,645,731; WO 02/077604; WO03/065042; and WO 03/019141) is used in the present invention to correlate the statistical survivability of an individual patient based on a threshold level of greater than or equal to 5 tumor cells in 7.5 to 30 ml blood (1 to 2 tumor cells correspond to about 3000 to 4000 total tumor cells in circulation for a given individual):

Using this diagnostic tool, a blood sample from a cancer patient (WO 03/018757) is incubated with magnetic beads, coated with antibodies directed against an epithelial cell surface antigen as for example EpCAM, as disclosed in WO99/41613, US2002/0172987, Kagan et al., J. Clin. Ligand Assay 25: 104-110 (2002) and Witzig et al., Clin. Canc. Res. 8: 1085-1091 (2002). After labeling with anti-EpCAM-coated magnetic nanoparticles, the magnetically labeled cells are then isolated using a magnetic separator. The immunomagnetically enriched fraction is further processed for downstream immunocytochemical analysis or image cytometry, for example, in the Cell Spotter^{®} System (Immunicon Corp., PA). The magnetic fraction can also be used for downstream immunocytochemical analysis, RT-PCR, PCR, FISH, flowcytometry, or other types of image cytometry.

The Cell Spotter® System utilizes immunomagnetic selection and separation to highly enrich and concentrate any epithelial cells present in whole blood samples. The captured cells are detectably labeled with a leukocyte specific marker and with one or more tumor cell specific fluorescent monoclonal antibodies to allow identification and enumeration of the captured CTC's as well as unequivocal instrumental or visual differentiation from contaminating non-target cells. At an extraordinary sensitivity of 1 or 2 epithelial cells per 7.5- 30 ml of blood, this assay allows tumor cell detection even in the early stages of low tumor mass. The embodiment of the present invention is not limited to the Cell Spotter® System, but includes any isolation and imaging protocol of comparable sensitivity and specificity.

Currently available prognostic protocols have not demonstrated a consistently reliable means for correlating CTC's to predict progression free- or overall survival in patients with cancers such as metastatic breast cancer (MBC). Thus, there is a clear need for accurate detection of cancer cells with metastatic potential, not only in MBC but in metastatic cancers in general. Moreover, this need is accentuated by the need to select the most effective therapy for a given patient.

### Summary of the Invention

The present invention is a method for cancer prognosis, as defined in claim 1. It uses diagnostic tools, such as the Cell Spotter® System, in assessing time to disease progression, survival, and response to therapy based upon the absolute number, of circulating cancer cells (CTC's) in patients with metastatic cancer. The system immunomagnetically concentrates epithelial cells, fluorescently labels the cells, identifies and quantifies CTC's for positive enumeration. The statistical analysis of the cell count predicts survival. Prediction of survival is based upon a threshold comparison of the number of circulating tumor cells in blood with time to death and disease progression. Statistical analysis of long term follow-up studies of patients diagnosed with cancer established a threshold for the number of CTC found in blood and prediction of survival. An absence of CTC's is defined as fewer than 5 morphologically intact CTC's. The presence or absence of CTC's to predict survival is useful in making treatment choices. For example, the absence of CTC's in a woman previously untreated for metastatic breast cancer could be used to select hormonal therapy vs chemotherapy with less side effects and higher quality of life. In contrast, the presence of CTC's could be used to select chemotherapy which has higher side effects but may prolong survival more effectively in a high risk population. Thus, the invention has a prognostic role in the detection of CTC's in women with metastatic breast cancer.

### Brief Description of the Drawings

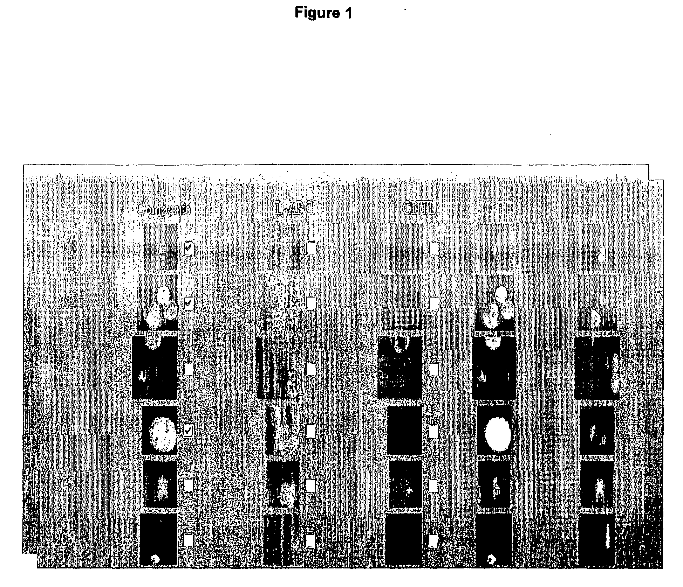

**Figure 1****:** Cell Spotter® fluorescent analysis profile used to confirm objects captured as tumor cells. Check marks signify a positive tumor cell based on the composite image. Composite images are derived from the positive selection for Epithelial Cell Marker (EC-PE) and for the nuclear dye (NADYE). A negative selection is also needed for the leukocyte marker (L-APC) and for control (CNTL).

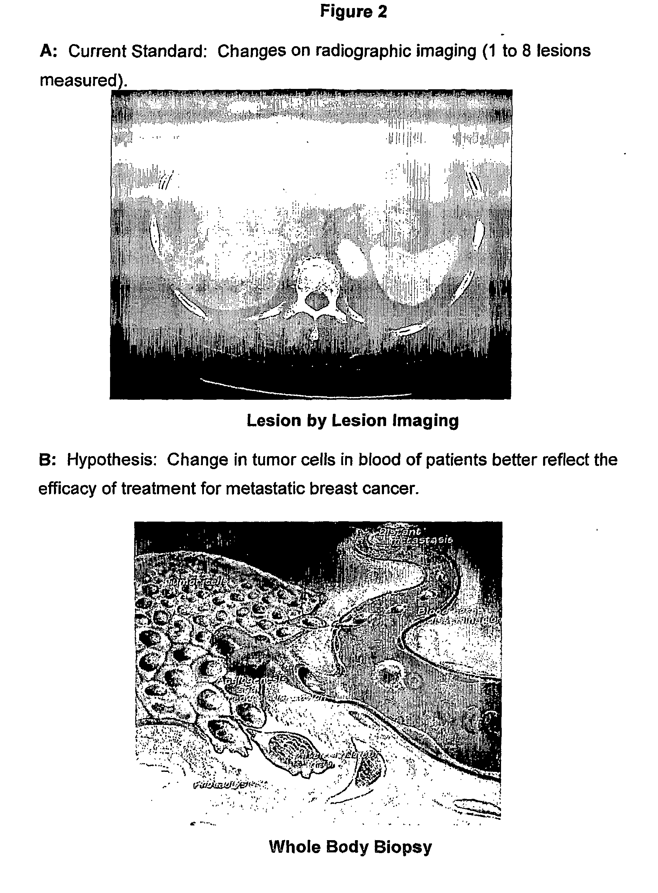

**Figure 2****:** Comparison of diagnostic methods to measure changes in tumor status. Shown is the current standard of physical measurement of discrete lesions using radiographic imaging (Panel A). A model illustrating the ability to assess changes in metastatic cancer burden by counting the numbers of CTC in blood is shown (Panel B).

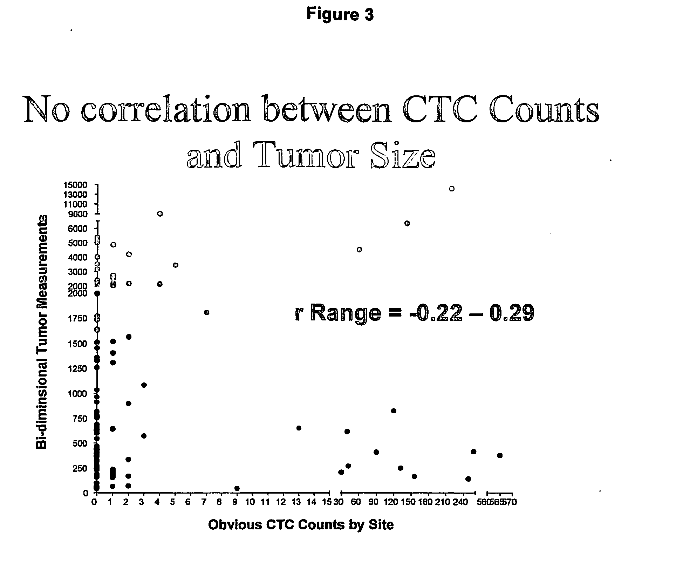

**Figure 3****:** Lack of correlation between the number of CTC's and tumor size in 69 patients.

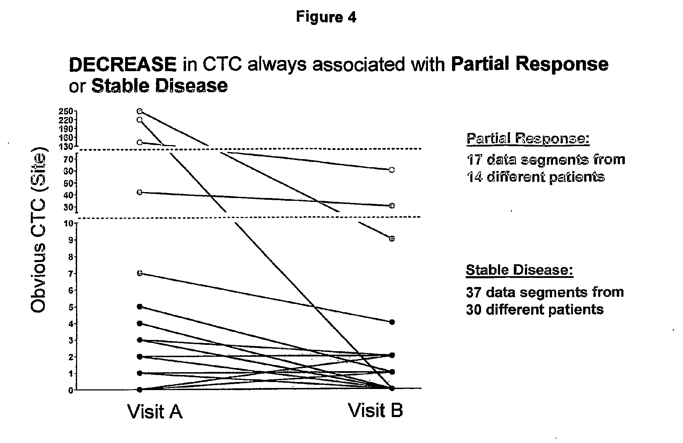

**Figure 4****:** Changes in the numbers of CTC's in patients with a partial response to therapy or with a stable disease state. CTC's either decreased or remained undetectable in all cases.

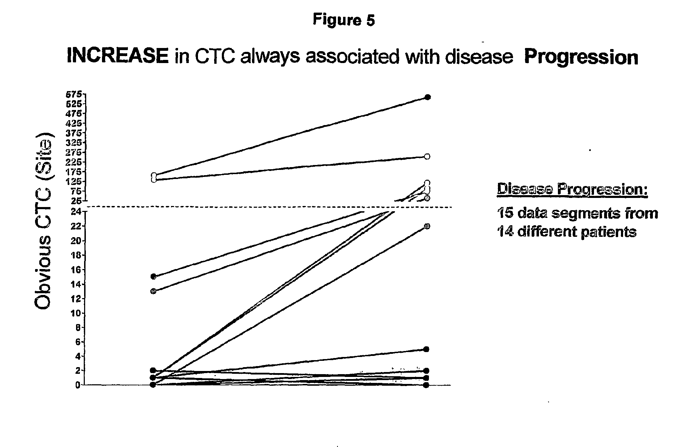

**Figure 5****:** Changes in the numbers of CTC's in patients with disease progression. CTCs either increased or remained undetectable with disease progression.

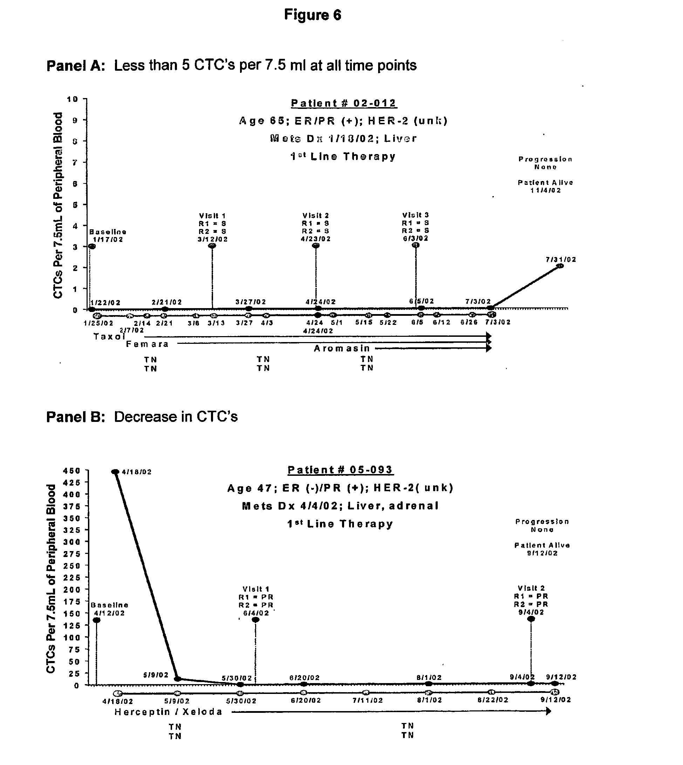

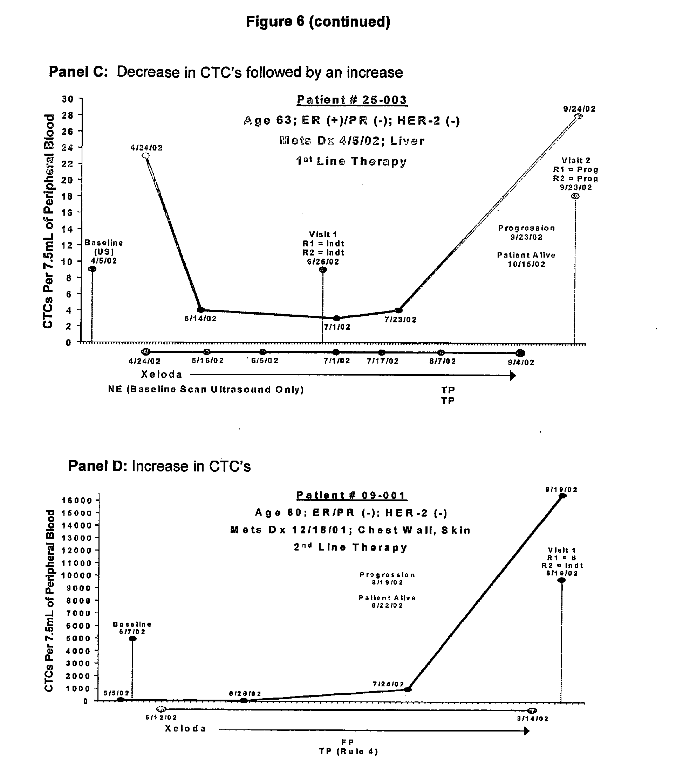

**Figure 6****:** Patient trends in the number of CTC's. Panel A shows a typical patient with less than 5 CTC's per 7.5 ml of blood. Panel B shows a typical patient with a decrease in CTC's during the course of therapy. Panel C shows a typical patient with a decrease in CTC's followed by an increase. Panel D shows a typical patient with an increase in CTC's.

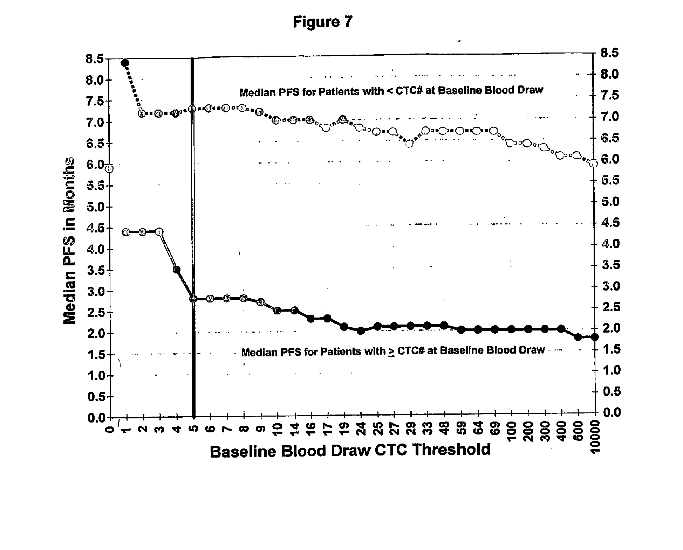

**Figure 7****:** Determination of an optimal CTC cutoff for distinguishing MBC patients with rapid progression. Analysis was performed using the CTC numbers obtained at baseline from the 102 patients included in the training set. Median PFS of patients with greater than or equal to the selected number of CTC in 7.5mL of blood is indicated by the solid line and median PFS of patients with less than the selected CTC level is indicated by the dashed line. Median PFS decreased as CTC increased and reached a plateau that leveled off at 5 CTC (indicated by the vertical line). The black dot indicates the median PFS of ∼5.9 months for all 102 patients. The selected cutoff of ≥5 CTC/7.5mL was used in all subsequent analyses.

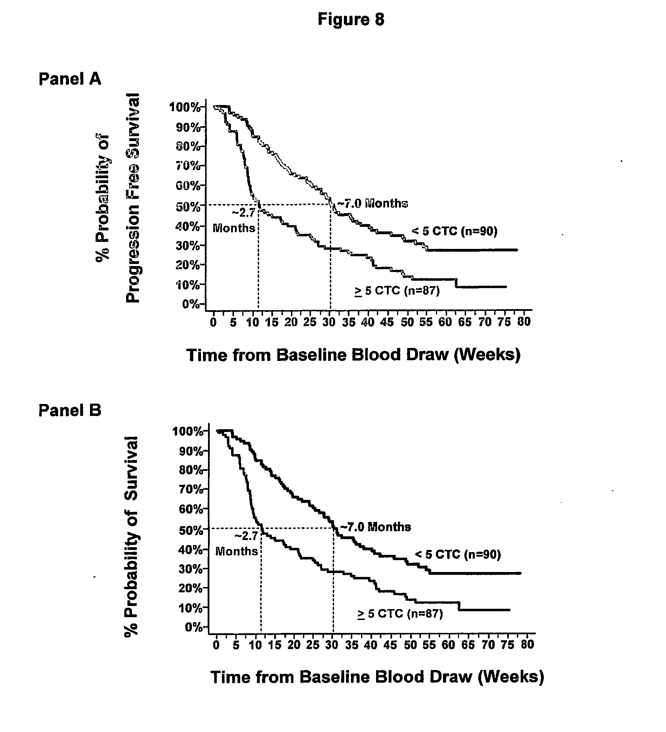

**Figure 8****:** The predictive value of baseline CTC for PFS and OS. Probabilities of PFS and OS of MBC patients with <5 (black line) and ≥5 (gray line) CTC's in 7.5mL of blood using the baseline blood draw prior to initiation of a new line of therapy are shown. PFS and OS were calculated from the time of the baseline blood draw.. Panel A: the probability of PFS using the baseline CTC count (n=177, log-rank p=0.0001, CoxHR =1.95, chi² =15.33, p= 0.0001). Panel B: the probability of OS using the baseline CTC count (n=102, log-rank p=0.0003, CoxHR =3.98, chi² =12.64, p= 0.0004).

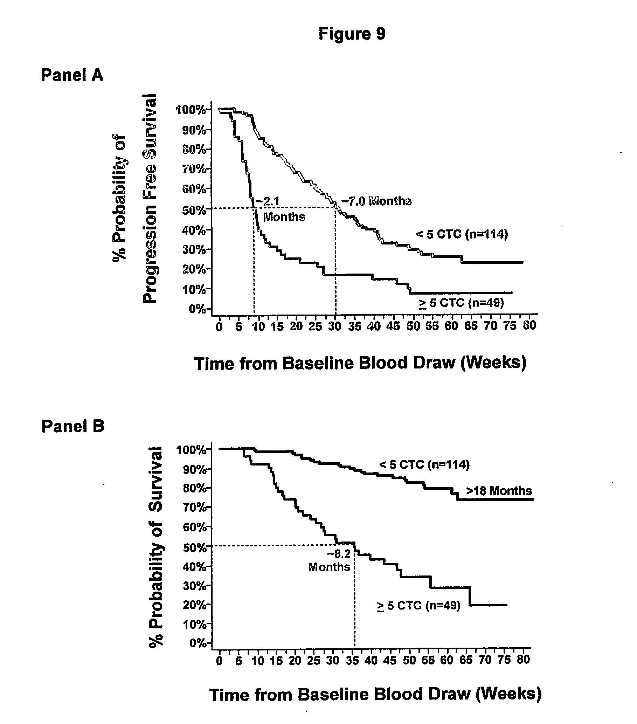

**Figure 9****:** The predictive value of CTC at the first follow-up for PFS and OS. Probabilities of PFS and OS of MBC patients with <5 (black line) and ≥5 (gray line) CTC's in 7.5mL of blood at the first follow-up after initiation of a new line of therapy are shown. PFS and OS were calculated from the time of the baseline blood draw. Panel A: the probability of PFS using the first follow-up blood draw (n=163, log-rank p<0.0001, CoxHR =2.73, chi² =25.25, p<0.0001). Panel B: the probability of OS using the first follow-up blood draw (n=68, log-rank p=0.0001, CoxHR =6.12, chi² =13.24, p= 0.0003).

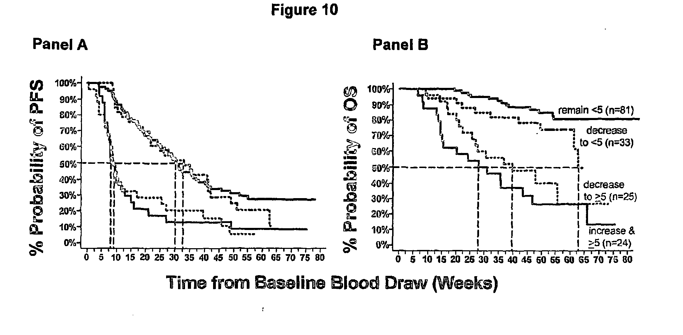

**Figure 10****:** A reduction in CTC count to below 5 at first follow-up blood draw after initiation of therapy (-4-5 weeks) predicts improved median PFS and OS. For both panels, the solid black line shows < 5 CTC's at both the baseline and first follow-up blood draws. The dotted black line shows ≥ 5 CTC's initially at baseline but decreasing to below 5 CTC's at the first follow-up. The dotted gray line shows ≥ 5 CTC's both at baseline and after follow-up, and the solid gray line shows an increase in CTC's from baseline level with ≥ 5 CTC's at follow-up. Panel A depicts the probability of PFS in patients while Panel B shows the probability of OS.

### Detailed Description of the Invention

The object of this invention provides for the detection of circulating tumor cells as an early prognostic indicator of patient survival. The method combines immunomagnetic enrichment technology, and immunofluorescent labeling technology with an appropriate analytical platform after initial blood draw. The associated test has the sensitivity and specificity to detect these rare cells in a sample of whole blood and to investigate their role in the clinical course of the disease in malignant tumors of epithelial origin. From a sample of whole blood, rare cells are detected with a sensitivity and specificity to allow them to be collected and used in the diagnostic assays of the invention, namely predicting the clinical course of disease in malignant tumors.

With this technology, circulating tumor cells (CTC) have been shown to exist in the blood in detectable amounts. This created a tool to investigate the significance of cells of epithelial origin in the peripheral circulation of cancer patients (Racila E., Euhus D., Weiss A.J., Rao C., McConnell J., Terstappen L.W.M.M. and Uhr J.W., Detection and characterization of carcinoma cells in the blood, Proc. Natl. Acad. Sci. USA, 95:4589-4594 (1998)). This study demonstrated that these blood-borne cells might have a significant role in the pathophysiology of cancer. Having a detection sensitivity of 1 epithelial cell per 5 ml of blood, the assay incorporates immunomagnetic sample enrichment and fluorescent monoclonal antibody staining followed by flowcytometry for a rapid and sensitive analysis of a sample. The results show that the number of epithelial cells In peripheral blood of eight patients treated for metastatic carcinoma of the breast correlate with disease progression and response to therapy. In 13 of 14 patients with localized disease, 5 of 5 patients with lymph node involvement and 11 of 11 patients with distant metastasis, epithelial cells were found in peripheral blood. The number of epithelial cells was significantly larger in patients with extensive disease.

The assay was further configured to an image cytometric analysis such that the immunomagnetically enriched sample is analyzed by the Cell Spotter® System (see Example 1). This is a fluorescence-based microscope image analysis system, which in contrast with flowcytometric analysis permits the visualization of events and the assessment of morphologic features to further identify objects.

The term Cell Spotter® System refers to an automated fluorescence microscopic system for automated enumeration of isolated cells from blood. The system contains an integrated computer controlled fluorescence microscope and automated stage with a magnetic yoke assembly that will hold a disposable sample cartridge. The magnetic yoke is designed to enable ferrofluid-labeled candidate tumor cells within the sample chamber to be magnetically localized to the upper viewing surface of the sample cartridge for microscopic viewing. Software presents suspect cancer cells, labeled with antibodies to cytokeratin and having epithelial origin, to the operator for final selection..

While isolation of tumor cells for the Cell Spotter® System can be accomplished by any means known in the art, one embodiment uses the Immunicon CellPrep^{™} System for isolating tumor cells using 7.5 ml of whole blood. Epithelial cell-specific magnetic particles are added and incubated for 20 minutes. After magnetic separation, the cells bound to the immunomagnetic-linked antibodies are magnetically held at the wall of the tube. Unbound sample is then aspirated and an isotonic solution is added to resuspend the sample. A nucleic acid dye, monoclonal antibodies to cytokeratin (a marker of epithelial cells) and CD 45 (a broad-spectrum leukocyte marker) are incubated with the sample. After magnetic separation, the unbound fraction is again aspirated and the bound and labeled cells are resuspended in 0.2 ml of an isotonic solution. The sample is suspended in a cell presentation chamber and placed in a magnetic device whose field orients the magnetically labeled cells for fluorescence microscopic examination in the Cell Spotter® System. Cells are identified automatically in the Cell Spotter® System and candidate circulating tumor cells presented to the operator for checklist enumeration. An enumeration checklist consists of predetermined morphologic criteria constituting a complete cell (see example 1).

The diagnostic potential of the Cell Spotter® System, together with the use of intact circulating tumor cells as a prognostic factor in cancer survival, can provide a rapid and sensitive method for determining appropriate treatment. Accordingly in the present invention, a method is provided for the rapid enumeration and characterization of tumor cells shed into the blood in metastatic and primary patients for prognostic assessment of survival potential.

The methods of the invention are useful in assessing a favorable or unfavorable survival, and even preventing unnecessary therapy that could result in harmful side-effects when the prognosis is favorable. Thus, the present invention can be used for prognosis of any of a wide variety of cancers, including without limitation, solid tumors and leukemia's including highlighted cancers: apudoma, choristoma, branchioma, malignant carcinoid syndrome, carcinoid heart disease, carcinoma (i.e. Walker, basal cell, basosquamous, Brown-Pearce, ductal, Ehrlich tumor, Krebs 2, merkel cell, mucinous, non-small cell lung, oat cell, papillary, scirrhous, bronchiolar, bronchogenic, squamous cell, and transitional cell), histiocytic disorders, leukemia (i.e. B-cell, mixed-cell, null-cell, T-cell, T-cell chronic, HTLV-II-associated, lymphocytic acute, lymphocytic chronic, mast-cell, and myeloid), histiocytosis malignant, Hodgkin's disease, immunoproliferative small, non-Hodgkin's lymphoma, plasmacytolma, reticuloendotheliosis, melanoma, chondroblastoma, chondroma, chondrosarcoma, fibroma, fibrosarcoma, giant cell tumors, histiocytoma, lipoma, liposarcoma, mesothelioma, myxoma, myxosarcoma, osteoma, osteosarcoma, Ewing's sarcoma, synovioma, adenofibroma, adenolymphoma, carcinosarcoma, chordoma, craniopharyngioma, dysgerminoma, hamartoma, mesenchymoma, mesonephroma, myosarcoma, ameloblastoma, cementoma, odontoma, teratoma, thymoma, trophoblastic tumor, adenocarcinoma, adenoma, cholangioma, cholesteatoma, cylindroma, cystadenocarcinoma, cystadenoma, granulose cell tumor, gynandroblastoma, hepatoma, hidradenoma, islet cell tumor, icydig cell tumor, papilloma, sertoli cell tumor, theca cell tumor, leiomyoma, leiomyosarcoma, myoblastoma, myoma, myosarcoma, rhabdomyoma, rhabdomyosarcoma, ependymoma, ganglioneuroma, glioma, medulloblastoma, meningioma, neurilemmoma, neuroblastoma, neuroepithelioma, neurofibroma, neuroma, paraganglioma, paraganglioma nonchromaffin, angiokeratoma, angiolymphoid hyperplasia with eosinophillia, angioma sclerosing, angiomatosis, glomangioma, hemangioendothelioma, hemangioma, hemangiopericytoma, hemangiosarcoma, lymphangioma, lymphangiomyoma, lymphangiosarcoma, pinealoma, carcinosarcoma, chondroscarcoma, cystosarcoma, phyllodes, fibrosarcoma, hemangiosarcoma, leiomyosarcoma, leukosarcoma, liposarcoma, lymphangiosarcoma, myoswarcoma, myxosarcoma, ovarian carcinoma, rhabdomyosarcoma, sarcoma (i.e. Ewing's experimental, Kaposi's and mast-cell), neoplasms (i.e. bone, breast, digestive system, colorectal, liver, pancreatic, pituitary, testicular, orbital, head and neck, central nervous system, acoustic, pelvic, respiratory tract, and urogenital, neurofibromatosis, and cervical dysplasia.

The following examples illustrate the predictive and prognostic value of CTC's in blood from patients. Note, the following examples are offered by way of illustration and are not in any way intended to limit the scope of the invention.

### • Example 1

### Enumeration of circulating cytokeratin positive cells using CellPrep^{™}

Cytokeratin positive cells are isolated by the CellPrep™ System using a 7.5 ml sample of whole blood. Epithelial cell-specific immunomagnetic fluid is added and incubated for 20 minutes. After magnetic separation for 20 minutes, the cells bound to the immunomagnetic-linked antibodies are magnetically held at the wall of the tube. Unbound sample is then aspirated and an isotonic solution is added to resuspend the sample. A nucleic acid dye, monoclonal antibodies to cytokeratin (a marker of epithelial cells) and CD 45 (a broad-spectrum leukocyte marker) are incubated with the sample for 15 minutes. After magnetic separation, the unbound fraction is again aspirated and the bound and labeled cells are resuspended in 0.2 ml of an isotonic solution. The sample is suspended in a cell presentation chamber and placed in a magnetic device whose field orients the magnetically labeled cells for fluorescence microscopic examination in the Cell Spotter® System. Cells are identified automatically in the Cell Spotter® System; control cells are enumerated by the system, whereas the candidate circulating tumor cells are presented to the operator for enumeration using a checklist as shown (Figure 1).

### • Example 2

### Assessment of the tumor load: Comparison between radiographic image analysis and the absolute number of CTC's.

Radiographic measurements of metastatic lesions are currently used to assess tumor load in cancer patients with metastatic disease. In general, the largest lesions are measured and summed to obtain a tumor load. An example of a bidimensional measurement of a liver metastasis in a breast cancer patient is illustrated in Figure 2A. A model depicting the necessity for measuring tumor load in the blood stream is illustrated in Figure 2B as a measurement of the actual active tumor load, and thus a better measure of the overall activity of the disease. To determine whether or not the absolute number of CTC's correlated with the dimension of the tumor measured by imaging a prospective study in patients with MBC was performed.

The Cell Spotter® System was used to enumerate CTC's in 7.5 ml of blood from 69 patients with measurable MBC. Tumor load was assessed by bidimensional radiographic measurements of up to 8 measurable lesions before initiation of therapy. The tumor load was determined by addition of the individual measurements (mm²). CTC's were enumerated in blood drawn before initiation of therapy.

Figure 3 shows the number of CTC's in 7.5 ml versus the bidimensional sums of tumor measurements in the 69 patients. From Figure 3, there is no correlation between the size of the tumor and the absolute number of tumor cells in the blood. Some patients with large tumors as measured by imaging have low numbers of CTC's and vice versa.

Thus, tumor burden as measured by radiographic imaging does not correlate with the absolute number of tumor cells present in the blood.

### • Example 3

### Assessment of the tumor load: Comparison between changes in the radiographic image and changes in the absolute number of CTC's.

Radiographic imaging is the current standard to assess whether a particular disease is responding, stabilizing, or progressing to treatment. The interval between radiographic measurements must be at least 3 months in order to give meaningful results. Consequently, a test that could predict response to therapy earlier during the treatment cycle would improve the management of patients treated for metastatic diseases, potentially increase quality of life and possibly improve survival. In this study, patients starting a new line of treatment for MBC were assessed to determine whether a change in the number of CTC's correlated with a change in patient status as measured by imaging.

The Cell Spotter® System was used to enumerate CTC's in 7.5 ml of blood in MBC patients about to start a new therapy, and at various time points during the treatment cycle. Radiographic measurements were made before initiation of therapy, 10-12 weeks after initiation of therapy and after completion of the treatment cycle (approximately 6 months after initiation of therapy), or at the time the patient progressed on therapy, whichever came first.

From image analysis, a partial response was found in 14 patients (17 data segments). CTC's either decreased or remained undetectable in all cases (see Figure 4). Stable disease by imaging was found in 30 patients (37 data segments). CTC's either decreased or remained not detectable in all cases (see Figure 4). Disease progression by imaging was found in 14 patients (15 data segments). CTC's increased in 7 of 15 cases. No CTC's were detected at either time point in the other 8 cases.

An increase in CTC's was only observed in patients with disease progression (100%). A decrease in CTC's was only observed in patients with a partial response or stable disease (100%). In patients with a partial response or stable disease, no CTC's were detected at both time points in 54 of 61 cases (89%).

### • Example 4

### Trends in the number of CTC's in patients treated for MBC as a guide to treatment.

A study in patients with MBC was performed to determine whether or not clear trends in changes of the number of CTC could be observed in patients treated for MBC, and whether or not simple rules could be applied to such trends in order to guide the treating physician in optimization of the treatment of patients with MBC.

The Cell Spotter® System was used to enumerate CTC's in 7.5 ml of blood. 81 patients, starting a new line of therapy for MBC, were enrolled in the study. CTC's were enumerated in blood drawn before initiation of therapy and at approximately every month thereafter.

Clear trends in the number of CTC's were observed in 76 of 81 (94%) patients. During the course of therapy, the number of CTC's was not detectable or remained below 5 CTC per 7.5 ml of blood in 50% of the patients. A typical example is shown in Figure 6A. The number of CTC's decreased during the course of therapy in 22% of the patients. A typical example is shown in Figure 6B. A decrease in the number of CTC's followed by an increase during the course of therapy was observed in 6% of the patients. A typical example is shown in Figure 6C. The number of CTC's increased during the course of therapy in 16% of the patients. A typical example is shown in Figure 6D. In 42 instances, 2 blood samples were prepared and analyzed at the time of each blood draw. Results using the first tubes drawn at the initial timepoint and the first tube drawn at the follow-up time point point were compared to results using the second tubes drawn at each timepoint. In only one of those cases, the change in the number of CTC's was different between the first tubes drawn and the second (or duplicate) tubes drawn (98% agreement). In this case, both tubes from the first blood draw had 0 CTC's, whereas for the second blood draw, one tube had 5 CTC(below the cut off) and the second tube had 6 CTC (above the cut off). In comparison to the reproducibility of CTC measurements, inter-reader variability of radiographic imaging when the same films were read by two different expert radiologists resulted in an agreement of only 81 %. More over, the agreement between the two radiologists in a set of 146 imaging segments was 85% when Progression versus non progression was measured and decreased to only 58% when Progression, Stable Disease and Partial response were measured. In contrast, analysis of CTC measurement was performed on the same data set by two different technologists, resulting in 100% agreement.

Thus, detection and monitoring CTC in patients treated for MBC is a more reproducible procedure to measure response to therapy than radiographic imaging.

### • Example 5

### Prediction of PFS and OS before initiation of therapy.

A study to correlate CTC levels before initiation of therapy with progression-free survival (PFS) and overall survival (OS) was performed whereby a threshold value of ≥5 CTC's/7.5 ml was used.

177 patients with measurable MBC were tested for CTC's in 7.5 ml of blood before starting a new line of treatment and at subsequent monthly intervals for a period of up to six months. Patients entering into any type of therapy and any line of therapy were included in the trial. Disease progression or response was assessed by the physicians at the sites for each patient.

As shown in Figure 7, median PFS decreased as CTC levels increased and reached a plateau that leveled off at 5 CTC's (vertical line). The median PFS was approximately 5.9 months for all patients (black dot). Based on the change in median PFS for positive patients and the Cox Hazard's ratio, a cutoff of ≥ 5 CTC's was used for all subsequent analysis.

Figure 8 shows a Kaplan Meier analysis of Progression Free Survival (PFS) and Overall Survival (OS) using the number of CTC measured in the baseline blood draws. In the 177 patients, the median PFS time was approximately 5.0 months. The patients with ≥ 5 CTC's/7.5 ml of blood at baseline had a significantly shorter PFS than patients with < 5 CTC's (approximately 2.7 months vs. 7.0 months, respectively). Overall survival (OS) reflected the same trend with a median OS of 10.1 months vs. > 18 months for patients with ≥ 5 CTC's vs. < 5 CTC's, respectively.

The measurement of the number of CTC prior to initation of a new line of therapy predicts the time until patients progress on their therapy, and predicts survival time. Because of this predictive ability, detection and measurement of CTC's at baseline provides information to physicians that will be useful in the selection of appropriate treatment. In addition, the ability to stratify patients into high and low risk groups in terms of PFS and OS may be very useful to select appropriate patients for entry into therapeutic trials. For novel drugs with potentially high toxicity, patients with poor prognostic factors may be the more appropriate target population. In contrast, drugs with minimal toxicity and promising therapeutic efficacy may be more appropriately targeted toward patients with favorable prognostic factors.

### • Example 6

### Prediction of PFS and OS after initiation of therapy.

A study to correlate CTC levels after initiation of therapy with progression-free survival (PFS) and overall survival (OS) was carried out using the number of CTC's at the first follow-up to predict PFS and OS..

163 patients with measurable MBC were evaluated for this analysis. Blood was drawn on average 4 weeks after the initiation of a new line of therapy. Disease progression or response was assessed by the physicians at the sites for each patient at an average time of 12 weeks after the initiation of therapy

As shown in Fig. 9, the 49 patients with ≥ 5 CTC's per 7.5 ml at first follow-up had a significantly shorter median PFS compared to the 114 patients with < 5 CTC's per 7.5 ml, approximately 2.1 months vs. 7.0 months, respectively. The same trend was observed for the median in overall survival, approximately 8.2 months for ≥ 5 CTC's and > 18 months for < 5 CTC's.

In a separate analysis, we compared two groups with known shorter or longer PFS and OS to the patients with decreasing CTCs. Specifically, patients whose CTCs were < 5 at baseline and at first follow up were known to have relatively long PFS and OS; i.e., this was a population with relatively good performance. Conversely, patients whose CTC rose from baseline to first follow-up with a CTC level of > 5 at first follow-up were known to have a relatively short PFS and OS; i.e., this was a population of patients with relatively poor performance. We then compared two additional groups of patients to these first two groups: first, patients whose CTC decreased from baseline to first follow-up to a level < 5. Second, we evaluated patients whose CTC decreased from baseline to first follow-up but the number of CTC at first follow-up was ≥ 5. Results are shown in Fig. 10. For the first control groups with < 5 CTC at baseline and first follow-up, the PFS and OS is relatively long, as expected. For the second control group with rising cells, the PFS and OS are relatively much shorter, again as expected. For the patients whose CTC decreased to < 5 at first follow-up, the PFS and OS approximated that of the patients who had < 5 CTC at both time points. In contrast, for patients whose CTC decreased but did not decrease to < 5, their prognosis was just as poor as those patients with rising CTCs.

Accordingly, CTC's must decline to below 5 at the first follow-up (approximately 4 weeks) to maximize PFS and OS, and to maximize the benefit associated with therapy.

### • Example 7

### Univariate and multivariate analysis of predictors of PFS and OS.

In order compare CTC levels with known parameters associated with PFS and OS, univariate and multivariate Cox proportional hazards regression analysis were performed. For predicting PFS, only the line of therapy, type of therapy and CTC levels at baseline and first follow-up were univariately significant. For OS, ER/PR status was also univariately significant where ER/PR is considered positive if either estrogen receptor, progesterone receptor, or both are positive. Patient status measured using the ECOG guidelines was also univariately significant for OS, where ECOG is the European Cooperative Oncology Group performance status, ranging from 0 to 5 (Table 2).

**Table 2. Univariate Cox regression analysis of independent parameters for prediction of PFS and OS.**

| | **Categories** | | **PFS** | | | | **OS** | | |
|---|---|---|---|---|---|---|---|---|---|
| **Parameter** | **Pos** | **Neg** | **# pat** | **HR** | **p-val** | **chi²** | **HR** | **p-val** | **chi²** |
| **Age (years)** | Age at Baseline | | 175 | 0.99 | 0.1099 | 2.56 | 0.99 | 0.1992 | 1.65 |
| **ECOG** | 2 vs. 1 vs. 0 | | 172 | 1.10 | 0.4465 | 0.58 | 1.63 | 0.0075 | 7.16 |
| **Stage** | 4 vs. 3 vs. 2 vs. 1 | | 164 | 0.94 | 0.5591 | 0.34 | 1.10 | 0.4746 | 0.51 |
| **ER/PR Status** | + | - | 175 | 0.85 | 0.3827 | 0.76 | 0.57 | 0.0253 | 5.00 |
| **Her2 Status** | 3 vs. 2 vs. 1 vs. | | 0148 | 0.90 | 0.1895 | 1.72 | 0.90 | 0.3557 | 0.85 |
| **Time to Metastasis** | Years | | 175 | 0.97 | 0.0252 | 5.01 | 0.92 | 0.0028 | 8.92 |
| **Line of Therapy** | ≥2^{nd} | 1^{st} | 175 | 1.68 | 0.0025 | 9.14 | 2.06 | 0.0042 | 8.19 |
| **Type of Therapy** | Chemo | Hormonal | 172 | 1.81 | 0.0016 | 9.97 | 3.46 | 0.0001 | 15.61 |
| **Baseline CTC** | ≥5 | <5 | 177 | 1.95 | 0.0001 | 15.33 | 4.39 | 0.0000 | 31.73 |
| **1^{st} follow-up CTC** | ≥5 | <5 | 163 | 2.73 | 0.0000 | 25.25 | 5.54 | 0.0000 | 38.02 |

| | | | | | | | | | |
|---|---|---|---|---|---|---|---|---|---|
| Stage = disease stage at primary diagnosis, Pos = positive, Neg = negative, Chemo = chemotherapy with or without other therapies, Horm. = hormonal therapy and/or immuno-therapy, HR = Cox hazards ratio. Age and time to metastasis were evaluated as continuous variables. | | | | | | | | | |

Stepwise Cox regression at a stringency level of p<0.05 to both include and exclude parameters was used separately for the baseline and first follow-up CTC levels to predict PFS and OS. Although some of the clinical factors maintained their relevance in the multivariate analysis, baseline CTC and persistent positive CTC at the first follow-up emerged as the strongest predictors of PFS and OS (Table 3).

**Table 3. Multivariate Cox regression analysis for prediction of PFS and OS using stepwise selection at a stringency level of p<0.06.**

| | **Categories** | | **PFS** | | | **OS** | | |
|---|---|---|---|---|---|---|---|---|
| **Parameter** | **Pos Neg** | | **HR** | **p-value** | **chi²** | **HR** | **p-value** | **chi²** |
| ***Analysis using Baseline CTC Count*** | | | | ***(n=172)*** | | ***(n=170)*** | | |
| **Baseline CTC** | ≥5 | <5 | 1.76 | 0.001 | 10.58 | 4.26 | 0.000 | 22.35 |
| **Line of Therapy** | ≥2^{nd} | 1^{st} | 1.73 | 0.002 | 9.75 | 2.38 | 0.001 | 10.32 |
| **Type of Therapy** | Chemo | Hormonal | 1.61 | 0.016 | 5.85 | 2.54 | 0.015 | 5.90 |
| **ECOG** | 2 vs. 1 vs. 0 | | ns | ns | ns | 1.48 | 0.024 | 5.10 |
| **Time to Metastasis** | Time in Years | | ns | ns | ns | 0.92 | 0.028 | 4.82 |
| | | | | | | | | |
| ***Analysis using 1^{st} Follow-Up CTC Count*** | | | | ***(n=162)*** | | ***(n=160)*** | | |
| **1^{st} Follow-Up CTC** | ≥5 | <5 | 2.52 | 0.000 | 23.56 | 6.49 | 0.000 | 38.34 |
| **ER/PR Status** | + | - | ns | ns | ns | 0.35 | 0.001 | 11.19 |
| **Line of Therapy** | ≥2^{nd} 1^{st} | | 1.58 | 0.013 | 6.22 | 2.29 | 0.006 | 7.67 |
| **ECOG** | 2 vs. 1 vs. 0 | | ns | ns | ns | 1.53 | 0.025 | 5.05 |

Multivariate Cox regression analysis using a stepwise selection process was used to evaluate association with PFS and OS. A stringency level (p-value) of 0.05 was used to both include and exclude parameters in the multivariate analyses. Results for each parameter that demonstrated a statistically significant correlation to PFS and OS are summarized in the table. CTC number was the strongest predictor of PFS and OS.
Stage = disease stage at primary diagnosis, Pos = positive
Neg = negative, HR = Cox hazards ratio, ns= not significant in multivariate analysis

### • Example 8

### Assessing response to therapy based upon CTC's after first follow-up.

A study in patients with MBC was performed to determine whether or not the number of CTC's after the first follow-up provided a relevant index for assessing response to therapy.

The Cell Spotter® System was used to enumerate CTC's in 7.5 ml of blood. 163 clinically diagnosed metastatic breast patients were compared for CTC's at the first follow-up blood draw which averaged 4.5 ±2.4 weeks (median 4.0 weeks, ranging from 1.4 to 16.9 weeks) from the time of the baseline blood draw. CTC's were enumerated in blood drawn before initiation of therapy and at approximately every month thereafter. Using a threshold value of less than 5 CTC's per 7.5 ml of blood, CTC counts at first follow-up were compared with patient clinical status, such that patients with stable or responding disease were categorized as no progression, and patients with clinical disease progression based upon bidimensional imaging determination from the baseline and first follow-up were categorized as Progression (See Table 4).

**Table 4: Enumeration of metastatic breast cancer patients at first follow-up blood draw.**

| **Imaging determination** | **Patients with <5 CTC's after 1^{st} follow-up** | **Patients with ≥5 CTC's after 1^{st} follow-up** | **Total** |
|---|---|---|---|
| Stable or Responding (no progression) | 94 | 14 | 108 |
| Progression | 20 | 35 | 55 |
| Total | 114 | 49 | 163 |

94 patients having less than 5 CTC's when assayed at the first follow-up showed no disease progression, showing agreement between CTC counts and response to therapy. 35 patients having 5 or more CTC's when assayed at the first follow-up showed disease progression, again showing agreement between CTC numbers at first follow-up and lack of response the therapy. 20 patients showed less than 5 CTC's with disease progression, which represented false negative results. These results would not be clinically harmful because these patients would continue to receive therapy as they would have without the use of CTC analysis. However, 14 patients showed ≥ 5 CTCs at first follow-up with no radiographic evidence of Progression, indicating false positive responses. While these responses might result in changing therapy in a patient that may benefit from that therapy, the new therapy would be expected to be helpful in thesepatients, and the number of false positives is acceptable low. Thus, overall, the enumeration of CTC's at the first follow-up gave an indication of the response to therapy in 129 of 163 patients evaluated..

## Claims

1. A method for survival prognosis in a patient comprising:
a) immunologically enriching a fraction of a blood specimen of said patient, said specimen comprising a mixed cell population suspected of containing circulating tumor cells (CTCs), wherein said enrichment step comprises mixing said specimen with magnetic particles coupled to an antibody which specifically binds to EpCAM and subjecting said specimen-magnetic particle mixture to a magnetic field to produce a cell suspension enriched in magnetic particle-bound CTCs;
b) confirming the structural integrity of cytokeratin-positive CTCs to be intact, wherein cytokeratin-positive cells are identified by labeling with a monoclonal antibody to cytokeratin, and wherein said structural integrity is determined through fluorescence-based analysis of morphologic features; and
c) measuring the number of said intact CTCs to determine patient survival, wherein a CTC number above or equal to five CTCs per 7.5 ml of blood is indicative of a lower said survival prognosis.

2. A method as claimed in claim 1, wherein said survival prognosis is determined for metastatic breast cancer patients, metastatic prostate cancer patients and/or metastatic colon cancer patients.

## Patentansprüche

1. Verfahren zur Überlebensprognose bei einem Patienten, das Folgendes umfasst:
a) immunologisch Anreichern einer Fraktion einer Blutprobe des Patienten, wobei die Probe eine gemischte Zellpopulation umfasst, von der angenommen wird, dass sie zirkulierende Tumorzellen (CTCs) enthält, wobei der Anreicherungsschritt ein Mischen der Probe mit magnetischen Partikeln umfasst, die an einen Antikörper gekoppelt sind, der spezifisch an EpCAM bindet, und ein Aussetzen der Probe/magnetische Partikel-Mischung einem magnetischen Feld, um eine Zellsuspension herzustellen, die mit an magnetische Partikel gebundene CTCs angereichert ist;
b) Bestätigen, dass die strukturelle Integrität von Cytokeratin-positiven CTCs intakt ist, wobei Cytokeratin-positive Zellen durch Markieren mit einem monoklonalen Antikörper gegen Cytokeratin identifiziert werden und wobei die strukturelle Integrität bestimmt wird durch eine fluoreszenzbasierte Analyse von morphologischen Merkmalen; und
c) Messen der Anzahl der intakten CTCs, um das Überleben des Patienten zu bestimmen, wobei eine CTC-Anzahl von fünf CTCs oder mehr pro 7,5 ml Blut auf eine weniger gute Überlebensprognose hinweist.

2. Verfahren wie in Anspruch 1 beansprucht, wobei die Überlebensprognose für Patienten mit metastasierendem Brustkrebs, Patienten mit metastasierendem Prostatakrebs und/oder Patienten mit metastasierendem Colonkrebs bestimmt wird.

## Revendications

1. Procédé pour le pronostic de survie d'un patient comprenant les étapes consistant à :
a) enrichir immunologiquement une fraction d'un spécimen de sang dudit patient, ledit spécimen comprenant une population de cellules mixtes suspectées de contenir des cellules tumorales en circulation (CTCs), où ladite étape d'enrichissement comprend le mélange dudit spécimen avec des particules magnétiques couplées à un anticorps qui se lie spécifiquement à EpCAM et la soumission dudit mélange spécimen-particules magnétiques à un champ magnétique pour produire une suspension de cellules enrichie en CTCs liées à des particules magnétiques ;
b) confirmer l'intégrité structurale de CTCs cytokératine-positives comme étant intactes, où les cellules cytokératine-positives sont identifiées par marquage avec un anticorps monoclonal à la cytokératine, et où ladite intégrité structurale est déterminée par analyse à base de fluorescence de caractéristiques morphologiques ; et
c) mesurer le nombre desdites CTC intactes pour déterminer la survie du patient, où un nombre de CTC supérieur ou égal à 5 CTC pour 7,5 mL de sang est indicatif d'un dit pronostic de survie inférieur.

2. Procédé selon la revendication 1, dans lequel ledit pronostic de survie est déterminé pour des patients atteints de cancer du sein métastatique, de patients atteints du cancer de la prostate métastatique et/ou de patients atteints du cancer du côlon métastatique.
